# EUROPÄISCHE PATENTSCHRIFT

(11) **EP 2 341 861 B1**
(45) Veröffentlichungstag und Bekanntmachung des Hinweises auf die Patenterteilung: **26.02.2014**
(21) Anmeldenummer: 09777405.3
(22) Anmeldetag: 23.07.2009
(51) Int. Cl.: A61B 18/14

(54) **VORRICHTUNG ZUR HERSTELLUNG VON ANASTOMOSEN SOWIE KOAGULATIONSELEKTRODE**
DEVICE FOR PRODUCING ANASTOMOSES AND COAGULATION ELECTRODE
DISPOSITIF D ÉTABLISSEMENT D ANASTOMOSES ET ÉLECTRODE DE COAGULATION

(30) Priorität: 19.08.2008 DE 102008038313; 22.09.2008 DE 102008048293
(43) Veröffentlichungstag der Anmeldung: 13.07.2011
(60) Teilanmeldung: 13191846.8
(73) Patentinhaber: ERBE Elektromedizin GmbH, 72072 Tübingen (DE)
(72) Erfinder: BRODBECK, Achim, 72555 Metzingen (DE); MITZLAFF, Lothar, P-8600-531 Lagos (PT)
(74) Vertreter: Bohnenberger, Johannes
(86) Internationale Anmeldenummer: PCT/EP2009/005365
(87) Internationale Veröffentlichungsnummer: WO 2010/020328

(56) Entgegenhaltungen:
- WO-A1-03/061487
- WO-A2-99/59486
- US-A- 3 774 615
- US-A1- 2006 020 263
- US-A1- 2007 276 363

## Beschreibung

Die Erfindung betrifft eine Vorrichtung zum Herstellen von Anastomosen gemäß dem Oberbegriff des Patentanspruches 1 sowie eine Koagulationselektrode gemäß dem Anspruch 11.

Eine Anastomose ist eine Verbindung zwischen zwei anatomischen Strukturen. Beispielsweise können durchtrennte Blutgefäße bei einer Organtransplantation mittels Anastomosen verbunden werden.

In der Chirurgie sind unterschiedliche Verfahren zur Herstellung derartiger Anastomosen bekannt. Beispielsweise kann die Verbindung unter Verwendung von Nahttechniken hergestellt werden. Des Weiteren existieren Ansätze, die eine organische Verbindung mittels Klebetechniken erlauben. Hierbei werden häufig Fibrinkleber eingesetzt, die eine sehr vorteilhafte Verbindung von Organen, insbesondere Hohlorganen ermöglichen. Problematisch ist jedoch die Verträglichkeit dieser Kleber, da sie thrömbogene und toxische Eigenschaften aufweisen können.

Klemmtechniken werden ebenfalls eingesetzt, um an geeigneter Stelle eine Verbindung herzustellen. Zur Unterstützung der Durchführung von Anastomosen werden verschiedene Hilfsmittel, wie Ringe, Manschetten (sogenannte Cuffs) oder Stents verwendet.

Nachteilig ist hierbei, dass diese Hilfsmittel in der Regel in dem zu verbindenden Hohlorgan verbleiben und dort Abstoßungsreaktionen hervorrufen können oder das Risiko von Thrombosen erhöhen.

Aus der WO 03/061487 A1 ist es bekannt, Hohlorgane durch die Applikation eines geeigneten Hochfrequenzstroms (HF-Stroms) zu verbinden. Bei der Applikation des HF-Stroms kommt es zu einem biologischen Verschweißen der Gewebestrukturen. Die Zellsubstanz gerinnt, wobei die Proteinstrukturen verkleben, so dass eine kontrollierte, sichere und schnelle Verbindung hergestellt werden kann. Zur Applikation eines HF-Stroms stellt die WO 03/061487 A1 ein Instrument mit einer inneren Hülse und einer äußeren Hülse bereit. Diese Hülsen umfassen jeweils eine Elektrode, die ringförmig ausgebildet ist und an die der HF-Strom angelegt wird. Es handelt sich also um ein bipolares HF-Instrument. Zur Verbindung der Enden eines Blutgefäßes wird das erste Ende durch die Innenhülse geführt und derart umgeschlagen, dass das Gewebe auf der Außenseite der Innenhülse zu liegen kommt. Das zweite Ende des Gefäßes wird über die Innenhülse und den darauf befindlichen Endabschnitt des Blutgefäßes gezogen. Die äußere Hülse lässt sich öffnen und über die Innenhülse und das darauf befindliche Gewebe stülpen. Die Elektroden sind an den Hülsen derart angeordnet, dass das erste Ende und das zweite Ende des Gefäßes zwischen den Elektroden an den Hülsen eingeklemmt ist. Bei einer Applikation des HF-Stroms durchströmt dieser das Gewebe und verschweißt die Enden.

Ein Problem der WO 03/061487 A1 besteht darin, dass zum Verschweißen oder Koagulieren von Hohlorganen mit unterschiedlichen Durchmessern eine Mehrzahl verschieden ausgestalteter Instrumente benötigt werden. Insbesondere muss das Spaltmaß zwischen Elektroden und Hülsen derart dimensioniert sein, dass es an die Durchmesser der Wandstärken angepasst ist.

Nur wenn die Elektroden optimal an den Gewebestrukturen anliegen, erreicht man eine vorteilhafte Verschweißung der Gewebestrukturen. Der Koagulationsvorgang erfordert während des gesamten Koagulationsvorgangs ein aufrechtzuerhaltendes Anpressen der Gewebestrukturen bzw. Gefäßendabschnitte.

Schließlich sind die mit dem Instrument der WO 03/061487 A1 erzielten Ergebnisse stark von der Auswahl eines passenden Instruments, d. h. vom "Augenmaß" des behandelnden Arztes abhängig und daher nur schwer zu dokumentieren und rekonstruieren.

Ausgehend von diesem Stand der Technik ist es Aufgabe der vorliegenden Erfindung, eine Vorrichtung zur Herstellung von Anastomosen zwischen einem ersten und einem zweiten Hohlorgan bereitzustellen, die einfach zu bedienen und zur Bildung von qualitativ hochwertigen Anastomosen geeignet ist und die vorab genannten Nachteile vermeidet. Des Weiteren soll eine hierfür geeignete Koagulationselektrode angegeben werden.

Die vorliegende Erfindung löst diese Aufgabe durch die Vorrichtung gemäß Anspruch 1 .

Insbesondere wird die Aufgabe durch eine Vorrichtung zur Herstellung von Anastomosen zwischen einem ersten und einem zweiten Hohlorgan gelöst, die jeweils eine Innen- und eine Außenfläche aufweisen, umfassend eine Hülse mit einer Innenelektrodenanordnung, über welche das erste Hohlorgan so umgestülpt wird, dass seine Innenfläche nach außen zu liegen kommt, und eine Außenelektrodenanordnung, die der Innenelektrodenanordnung insbesondere radial gegenüber liegt und mit dem zweiten Hohlorgan, welches über das erste Hohlorgan geschoben ist, so in elektrischen Kontakt bringbar ist, dass die Elektrodenanordnungen Außenflächen der Hohlorgane kontaktieren, wobei die Außenelektrodenanordnung eine Vielzahl von durch mindestens ein Führungsorgan gegeneinander bewegbaren Lamellen umfasst, die derart ausgebildet und beweglich angeordnet sind, dass Innenränder der Lamellen in einem Schließzustand eine Durchgangsöffnung bilden und am zweiten Hohlorgan anliegen und in einem Öffnungszustand ein von außen zur Durchgangsöffnung führender Spalt zwischen den Lamellen gebildet ist, durch den die miteinander verbundenen Hohlorgane aus der Durchgangsöffnung nach außen geführt werden können.

Ein wesentlicher Gedanke der vorliegenden Erfindung besteht also darin, dass die Außenelektrode durch eine Vielzahl von gegeneinander bewegbaren Lamellen gebildet wird. Die Lamellen sind derart angeordnet, dass sie zumindest zwei Zustände einnehmen können, einen Schließzustand und einen Öffnungszustand. In dem Öffnungszustand sind zumindest die Lamellen derart von der Hülse mit der Innenelektrodenanordnung beabstandet, dass sich eine Durchgangsöffnung ausbildet, in der die entsprechenden Abschnitte der Hohlorgane, vorzugsweise noch unter weitgehender Abwesenheit eines Gewebekontakts und radialer Kraftwirkung, positioniert werden können. In dem Schließzustand liegen die Innenränder der Lamellen zumindest abschnittsweise auf dem Hohlorgan auf und ermöglichen eine unmittelbare Applikation des HF-Stroms. Die Außenelektrodenanordnung mit den Lamellen ist teilbar. Im Öffnungszustand kann sich ein Spalt ausbilden, der die Lamellen teilt. Aufgrund des Spalts lässt sich das unverbundene Hohlorgan oder Gefäß einbringen bzw. das verbundene aus der Vorrichtung entfernen.

Vorzugsweise können die Lamellen einen mechanischen Druck auf die Hohlorgane ausüben. Die Schließ- und Koagulationsbedingungen lassen sich relativ genau bestimmen. Hierdurch kann die Bildung von Anastomosen mit einer hohen Belastbarkeit gewährleistet werden. Nach einer Verbindung der Hohlorgane lässt sich in dem Öffnungszustand die Vorrichtung leicht entfernen. Eine Verletzung der Organe oder eine übermäßige Belastung der hergestellten Verbindung wird vermieden.

Die Lamellen können derart einstellbar sein, dass im Schließzustand eine Öffnung entsteht, die dem zu behandelnden Hohlorgan entspricht. Somit können mit einer Vorrichtung gemäß der vorliegenden Erfindung Hohlorgane behandelt werden, die unterschiedliche Abmessungen aufweisen. Beispielsweise können Blutgefäße mit unterschiedlichen Durchmessern behandelt werden. Ein Instrument kann dafür geeignet sein, Venen und Arterien zu verbinden.

Die Lamellen können nach Art einer Irisblende aufgebaut und/oder angeordnet sein. Die Durchgangsöffnung ist somit im Wesentlichen kreisförmig aufgebaut. Dies ist für eine Vielzahl von Applikationen, beispielsweise bei der Verbindung von Gefäßen, vorteilhaft. Des Weiteren ist eine Irisblende besonders dazu geeignet, eine im Wesentlichen durchgehende Anlagefläche der Lamellen bereitzustellen. Somit kommt es zu einer durchgehenden Applikation des HF-Stroms. Ein applizierter mechanischer Druck kann regelmäßig über die Außen- und Oberfläche des zweiten Hohlorgans verteilt werden.

Die Hülse kann relativ zur Außenelektrode fixiert in einem Halter oder einer Halterung angeordnet sein. Somit befindet sich die Hülse in einer vorgegebenen Position gegenüber der Außenelektrode. Ein Positionieren der Hülse gegenüber der Außenelektrode wird somit entbehrlich. Des Weiteren kann die erfindungsgemäße Vorrichtung durch die Fixierung in einem Halter wesentlich kompakter aufgebaut sein. Die Handhabung erleichtert sich durch die feste oder einstellbare Positionierung.

Die Halterung kann zur individuellen Handhabung der Hülse abnehmbar sein. Das heißt, die Halterung lässt sich von der Vorrichtung lösen und getrennt von dieser bedienen. Somit ist es möglich in einer Vorbereitungsphase das erste Hohlorgan und/oder das zweite Hohlorgan in bzw. auf der Hülse anzuordnen. Danach können Hülse und Vorrichtung derart gekoppelt werden, dass sich die Hülse gegenüber den Außenelektroden in einer vorteilhaften Position befindet. Nach dem Koagulationsvorgang kann es vorteilhaft sein, die Halterung wieder von der Vorrichtung zu lösen, um die verbundenen Hohlorgane nicht unnötig zu strapazieren.

Die Innenränder der Lamellen können im Schließzustand zur Hülse einen im Wesentlichen gleichen Abstand aufweisen. Im Schließzustand liegen also die Innenränder auf dem zweiten Hohlorgan, insbesondere auf dessen Innenfläche auf. Der HF-Strom wird unmittelbar auf das Gewebe appliziert. Die erzielte Verbindung ist entsprechend hochwertiger.

Die Durchgangsöffnung kann hinsichtlich Ihres Durchmessers durch Bewegung der Lamellen einstellbar sein. Die Vorrichtung lässt sich also durch das Bewegen der Lamellen öffnen und schließen.

Der Spalt kann im Öffnungszustand die Außenelektrode in zwei Hälften teilen, die voneinander weg bewegbar sind. Somit ist es möglich, eine Irisblende zu schaffen, die sich beispielsweise in zwei Halbkreise aufteilen lässt. Das schonende Entfernen der Vorrichtung nach einem Koagulationsvorgang wird somit erleichtert.

Die Hülse kann an einer Betätigungseinrichtung gehalten und derart in zwei Teile zerlegbar ausgebildet sein, dass die Teile auch von einem Schließzustand zur Bildung eines geschlossenen Rohrabschnittes in einen Öffnungszustand zum Abnehmen vom Hohlorgan bringbar sind. Die Hülse kann also zweiteilig ausgebildet sein, so dass sich diese nach oder vor der Applikation des HF-Stroms zerlegen lässt. Beispielsweise kann es möglich sein, die einzelnen Teile der Hülse auseinanderzuspreizen, um eine bessere Handhabbarkeit der Organe zu gewährleisten. Auch kann das Aufspreizen der Hülse das Abgleiten des über die Hülse gestülpten Hohlorgans verhindern.

Die Betätigungseinrichtung kann in einem Schließzustand vorgespannt sein. Somit stellt sich der Schließzustand automatisch ein.

Die Lamellen können durch eine Spanneinrichtung in dem Schließzustand mit einer definierten Kraft vorgespannt sein. Vorzugsweise lässt sich die Spanneinrichtung derart einstellen, dass die Lamellen im Schließzustand mit einer vordefinierten Kraft gegen die Hohlorgane gedrückt werden. Diese Kraft ermöglicht nicht nur eine sichere Koagulation des Gewebes, sondern ist auch derart bemessen, dass es zu keiner Verletzung der Hohlorgane kommt. Durch das Einstellen der Spanneinrichtung kann eine Wiederholbarkeit der Behandlungsergebnisse sichergestellt werden.

Die oben aufgeführte Aufgabe wird ebenfalls durch eine Koagulationselektrode welche nicht Teil der Erfindung ist gelöst, wobei diese umfasst:
- einen Lagerring;
- eine Vielzahl von Lamellen, die am Lagerring von einem Öffnungszustand in einen Schließzustand nach Art einer Irisblende verschwenkbar gelagert sind, zur Bildung von Elektrodenabschnitten;
- einen Kulissenring, der gegenüber dem Lagerring verdrehbar angeordnet und mit Führungsorganen an den Lamellen derart in Eingriff steht, dass bei einem Verdrehen des Kulissenringes gegenüber dem Lagerring die Lamellen vom Schließzustand in den Öffnungszustand und umgekehrt verschwenkt werden, wobei der Lagerring und der Kulissenring jeweils derart zweigeteilt ausgebildet sind, dass sie zusammen mit den am Lagerring gelagerten bzw. mit dem Kulissenring in Eingriff stehenden Lamellen voneinander fortbewegbar sind.

Eine derartige Koagulationselektrode hat ähnliche Vorteile, wie die der oben beschriebenen Vorrichtung. Insbesondere ist die Koagulationselektrode teilbar und somit nach der Applikation des HF-Stroms leicht abnehmbar. Das heißt, die Koagulationselektrode lässt sich leicht von dem verbundenen Hohlorgan entfernen.

Der Kulissenring kann auch eine Lamelle sein und deren Funktionen übernehmen.

Der Kulissenring kann Kulissenringabschnitte umfassen, die jeweils getrennt voneinander, insbesondere durch Führungsstangen bewegbar sind.

Der Kulissenring kann genau zwei Kulissenringabschnitte umfassen, die den Kulissenring bilden. Die Abschnitte können zur Betätigung der Lamellen dienen. Insbesondere lassen sich die Lamellen mittels der Abschnitte gegenüber dem Lagerring (vorzugsweise umfassend zwei Lagerringabschnitte) verdrehen. Die Lamellen werden in den Öffnungs- bzw. Schließzustand gebracht.

Nachfolgend wird die Erfindung anhand von einigen Ausführungsbeispielen, die mittels Abbildungen näher erläutert werden, beschrieben.

Hierbei zeigen:
Fig. 1 ein elektrochirurgisches Instrument zur Herstellung von Anastomosen mit geöffneten Branchen;
Fig. 2 das Instrument aus Fig. 1 mit geschlossenen Branchen (Irisblende geöffnet);
Fig. 3 das Instrument aus Fig. 1 und 2 mit geschlossenen Branchen (Irisblende geschlossen),
Fig. 4 eine Schließkrafteinstellvorrichtung für das Instrument gemäß den Fig. 1 bis 3;
Fig. 5 einen Detailausschnitt der Irisblende aus Fig. 1 mit Positioniervorrichtung;
Fig. 6 die Irisblende aus Fig. 5 in einer Explosionsdarstellung;
Fig. 7 eine weitere Ausführungsform der Lamellen für eine Irisblende;
Fig. 8 eine Detailansicht der Irisblende aus Fig. 1 (Irisblende geschlossen);
Fig. 9 eine Detailansicht der Irisblende aus Fig. 1 (Irisblende geöffnet);
Fig. 10 eine Detailansicht auf eine weitere Ausführungsform einer Irisblende (Irisblende geschlossen);
Fig. 11 die Irisblende aus Fig. 10 (Irisblende geöffnet);
Fig. 12 eine weitere Ausführungsform der Irisblende in Explosionsansicht;
Fig. 13 und 14 Detailansichten auf die Irisblende aus Fig. 12.

In der nachfolgenden Beschreibung werden für gleiche und gleichwirkende Teile dieselben Bezugsziffern verwendet.

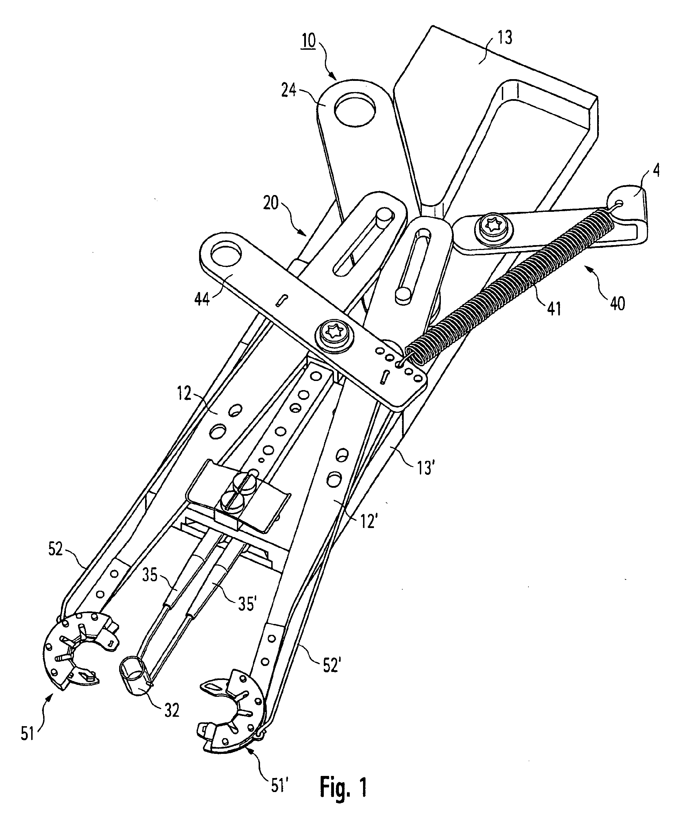

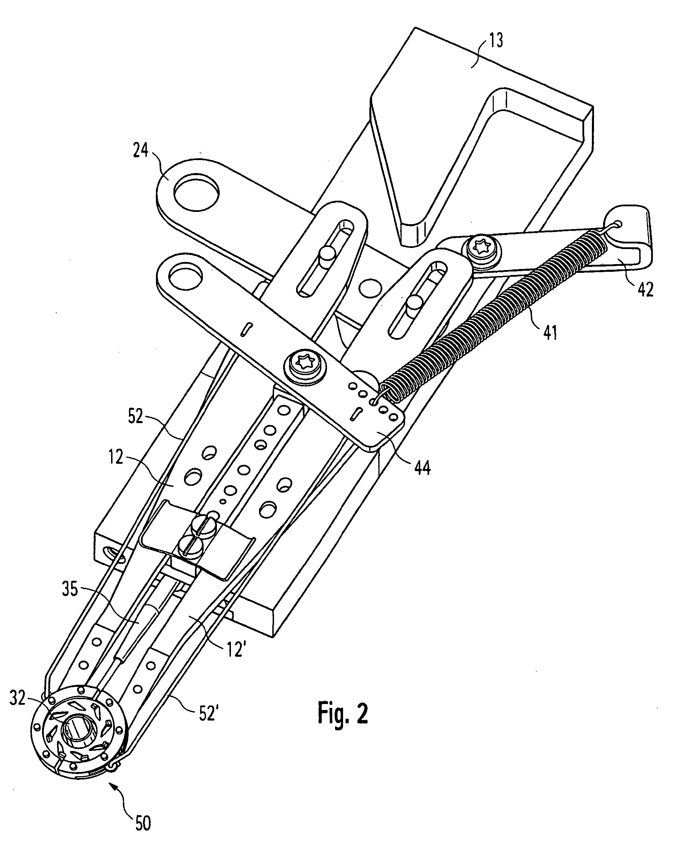

Fig. 1 zeigt ein elektrochirurgisches Instrument 10 zur Herstellung von Anastomosen. Das Instrument 10 umfasst eine Basisplatte 13, auf der Branchen 12, 12' drehbeweglich montiert sind. Eine Verschlussvorrichtung 20 dient zur Betätigung der Branchen 12, 12'. Diese umfasst ein Verschlussbetätigungselement 24, das über eine Kulissenführung mit den Branchen 12, 12' verbunden ist. Das Verschlussbetätigungselement 24 hat im Wesentlichen zwei Positionen, eine erste Position, in der das elektrochirurgische Instrument 10 geöffnet ist und eine zweite Position in der das elektrochirurgische Instrument 10 geschlossen ist. Vorzugsweise ist die Verschlussvorrichtung 20 derart ausgebildet, dass die Branchen 12, 12' in der zweiten Position fixiert sind, so dass ein unbeabsichtigtes Öffnen des elektrochirurgischen Instruments 10 nicht möglich ist (vgl. Fig. 2).

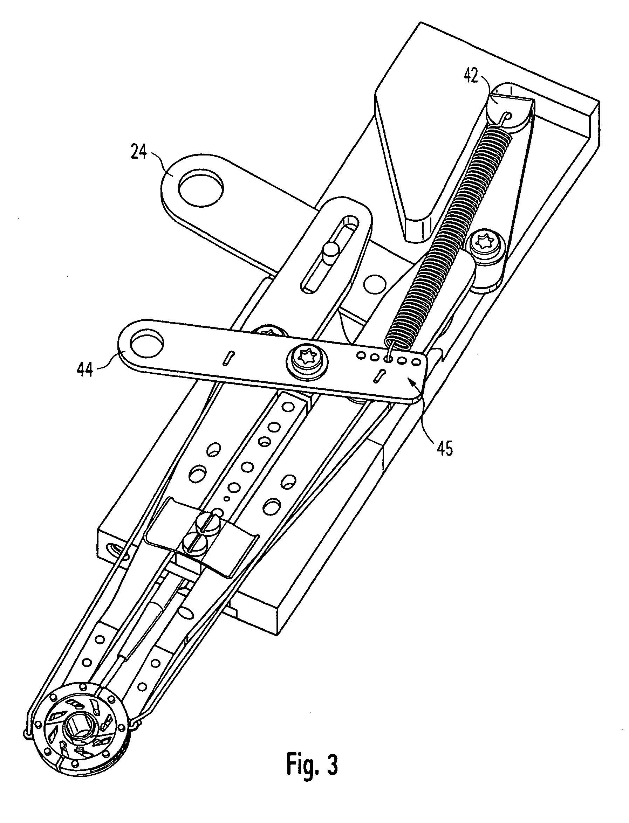

Am distalen Ende der Branchen 12, 12' befinden sich Halbblenden 51, 51', die im geschlossenen Zustand des elektrochirurgischen Instruments 10 (vgl. Fig. 2 und 3) eine Irisblende 50 ausbilden.

Des Weiteren weist das elektrochirurgische Instrument 10 eine Spannvorrichtung 40 zur Betätigung der Irisblende 50 auf. Diese Spannvorrichtung 40 umfasst ein Spannvorrichtungsbetätigungselement 42, einen Kraftumlenker 44, die drehbeweglich auf der Basisplatte 13 montiert sind, eine Spannfeder 41 und zwei Stellelemente 52, 52'. Die Stellelemente 52, 52' verlaufen jeweils entlang der ersten Branche 12 bzw. der zweiten Branche 12' und übertragen die mit der Spannvorrichtung 40 ausgeübten Kräfte auf die Mechanik der Irisblende 50. Die Spannvorrichtung 40 befindet sich in der geöffneten Position des elektrochirurgischen Instruments 10 ebenfalls in einer geöffneten Stellung (vgl. Fig. 1). In der zweiten, geschlossenen Position des elektrochirurgischen Instruments 10 kann die Spannvorrichtung 40 durch das Betätigen des Spannvorrichtungsbetätigungselements 42 gespannt werden. Fig. 3 zeigt das Spannvorrichtungsbetätigungselement 42 in gespanntem Zustand. Bei einer Betätigung des Spannvorrichtungsbetätigungselements 42 wird die Spannfeder 41, die mit dem Kraftumlenker 44 verbunden ist, gespannt. Es kommt zu einer Rotationsbewegung des Kraftumlenkers 44 um einen Drehpunkt, der zwischen den Branchen 12, 12' liegt. Das Gestänge bzw. die Stellelemente 52, 52', die im Wesentlichen punktsymmetrisch zu diesem Drehpunkt am Kraftumlenker 44 ansetzen, vollziehen eine Translationsbewegung in proximale (Stellelement 52') bzw. distale (Stellelement 52) Richtung. Diese Bewegung führt wie weiter unten näher erläutert zu einem Schließen und Öffnen der Irisblende 50.

Es sei angemerkt, dass die Spannfeder 41 in gespanntem Zustand über die Stellelemente 52, 52' durchgehend eine Kraft auf den Mechanismus der Irisblende 50 ausübt. Der Betrag dieser Kraft lässt sich durch eine Schließkrafteinstellvorrichtung 45 (vgl. Fig. 3) einstellen. Die Ausführungsform des Kraftumlenkers 44 gemäß der Fig. 3 umfasst hierfür eine Vielzahl von Bohrungen, die unterschiedliche Abstände zu dem Drehpunkt des Kraftumlenkers 44 aufweisen. Die Spannfeder 41 ist derart ausgebildet, dass sie sich in den einzelnen Bohrungen verankern lässt. Somit setzt die Federkraft der Spannfeder 41 je nach Position in den Bohrungen mit einem sich unterscheidenden Hebel an den Kraftumlenker 44 an. Wie in Fig. 1-3 gezeigt überragt der Kraftumlenker 44 die Basisplatte 15 derart, dass diese manuell betätigbar ist. Die Irisblende 50 lässt sich aber auch im nicht-gespannten Zustand über den Kraftumlenker 44, der als Betätigungselement dient, schließen und öffnen.

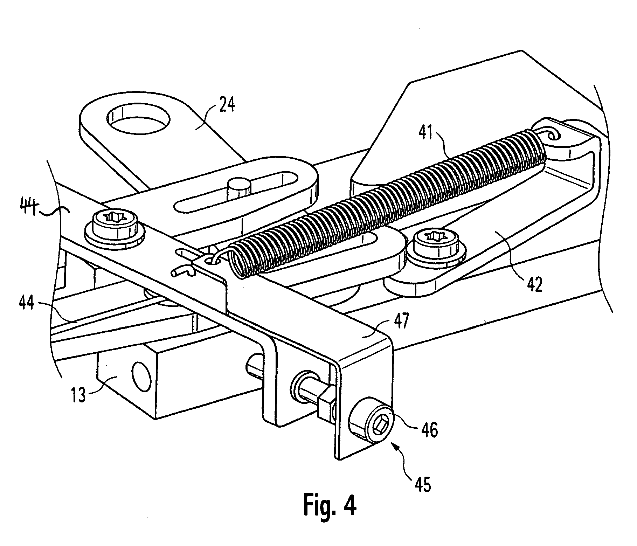

Fig. 4 zeigt eine weitere Ausführungsform der Spannvorrichtung 40. Hier umfasst die Schließkrafteinstellvorrichtung 45 eine Stellschraube 46 und einen Winkel 47. Der Winkel 47 ist derart an dem Kraftumlenker 44 befestigt, dass sich dieser durch ein Betätigen der Stellschraube 46 entlang einer Längsachse verschieben lässt. In dem in Fig. 4 gezeigten Ausführungsbeispiel setzt die Spannfeder 41 an dem Winkel 47 an. Durch ein Betätigen der Stellschraube 46 lässt sich der Hebel, mit dem die Spannfeder 41 Kraft auf den Kraftumlenker 44 ausübt, stufenlos einstellen.

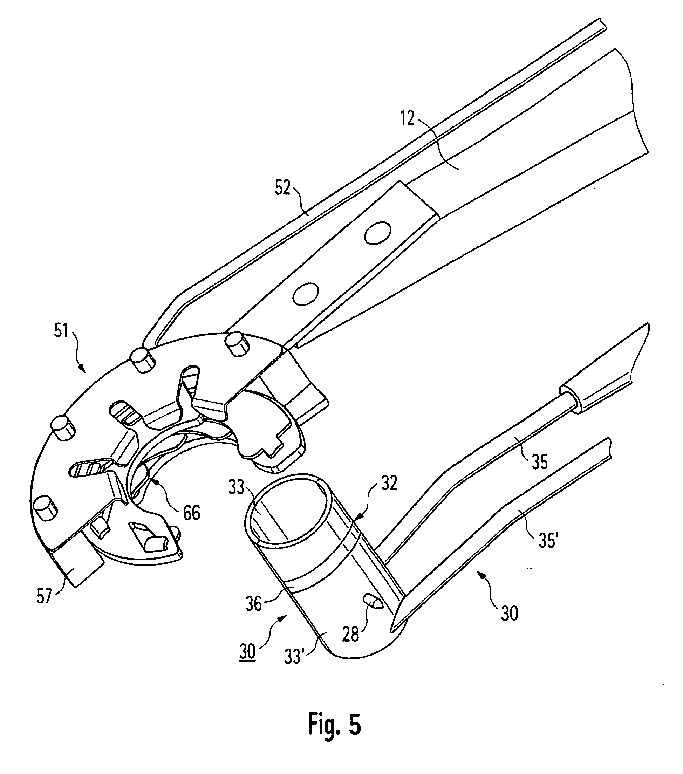

Ein Weiteres wesentliches Element des elektrochirurgischen Instruments 10 ist eine Positionierungsvorrichtung 30 (vgl. Fig. 5). Diese umfasst eine Hülse 32 zur Aufnahme der Hohlorgane und zwei Hülsenbetätigungsbranchen 35, 35'. Die Positionierungsvorrichtung 30 dient im Wesentlichen dazu, die Hohlorgane im Zentrum der Irisblende 50 zu positionieren. Des Weiteren stellt sie eine Hülsenelektrode 36 bereit. Die Hülse 32 ist im Wesentlichen zylindrisch ausgebildet und umfasst einen ersten Hülsenabschnitt 33 und einen zweiten Hülsenabschnitt 33'. Im Querschnitt, quer zur Längsachse der Hülse 32, bilden die Hülsenabschnitte 33, 33' jeweils einen Halbkreis. Die Hülsenabschnitte 33, 33' sind mit den Hülsenbetätigungsbranchen 35 bzw. 35' verbunden. Diese sind derart drehbeweglich an einem abnehmbaren Abschnitt 13' der Basisplatte 13 befestigt, dass sich die Hülsenabschnitte 33, 33' öffnen und schließen lassen. Im geschlossenen Zustand liegen die Hülsenabschnitte 33, 33' aneinander an und bilden die Hülse 32. Die Positionierungsvorrichtung 30 lässt sich also über die Hülsenbetätigungsbranchen 35, 35' öffnen und schließen. Bevorzugt sind die Hülsenbetätigungsbranchen 35, 35' mit einer Federkraft beaufschlagt, die die Hülsenabschnitte 33, 33' geschlossen hält. Der abnehmbare Abschnitt 13', auf dem Hülsenbetätigungsbranchen 35, 35' drehbeweglich gelagert sind, ist über eine Steckverbindung mit der Basisplatte 13 verbunden. Zur Aufnahme der Hohlorgane lässt sich die Positionierungsvorrichtung 30 von den übrigen Bestandteilen des elektrochirurgischen Instruments 10 ablösen und individuell handhaben.

Die Teilbarkeit der Hülse 32 ist beim Anlegen und Abnehmen des Instruments 10 an dem Hohlorgan von besonderem Vorteil. Um diesen Vorgang weiter zu erleichtern umfasst die Hülse 32 mindestens zwei Dorne 28, die radial zur Längsachse der Hülse 32 und auf den Hülsenabschnitten 33, 33' angeordnet sind. Wird das Hohlorgan oder dessen Abschnitte über die Hülse 32 geschoben, so fixieren die Dorne 28 die übergestülpten Hohlorgane oder Gefäße. Deren Vorschub wird durch die Hülsenbetätigungsbranchen 35, 35' begrenzt.

Nach der Aufnahme der Hohlorgane lässt sich der abnehmbare Abschnitt 13' wieder mit der Basisplatte 13 verbinden und in eine Position bringen, die dazu geeignet ist, die Abschnitte des Hohlorgans zu verbinden.

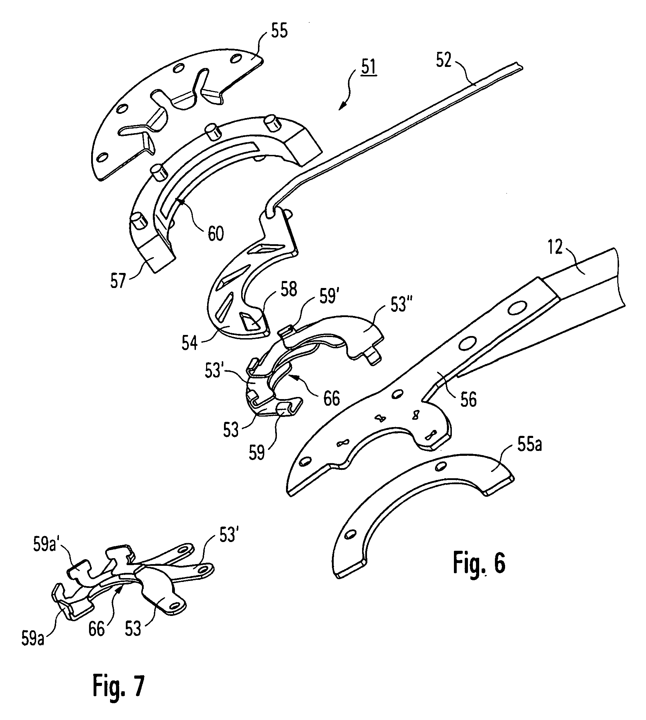

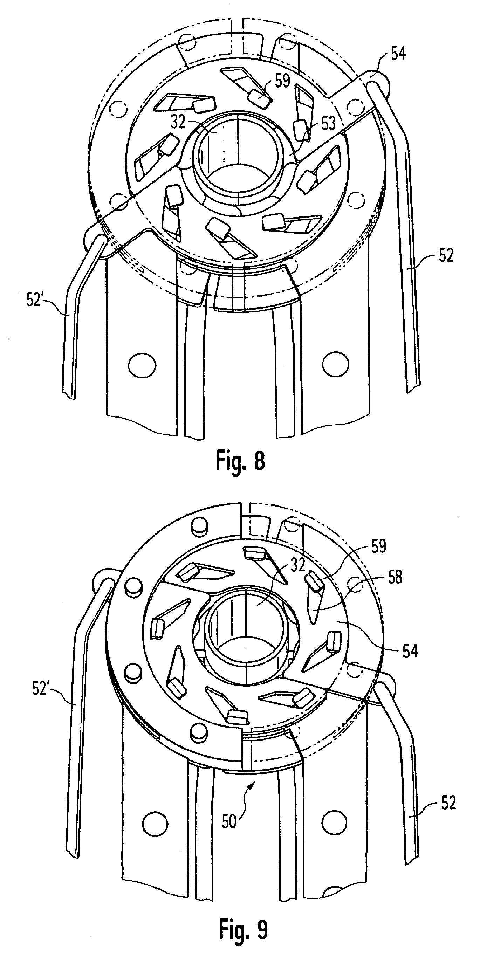

Ein wesentlicher Punkt der vorliegenden Erfindung besteht darin, den Gerätekopf des elektrochirurgischen Instruments 10 in Form einer teilbaren Irisblende aufzubauen. Wie in der Fig. 1 verdeutlicht, umfasst die Irisblende 50 zwei Halbblenden 51, 51', die im geschlossenen Zustand als eine Irisblende 50 zusammenwirken. Der Aufbau einer Halbblende, nämlich der Halbblende 51 ist in der Fig. 6 veranschaulicht. Diese umfasst eine Befestigungsplatte 56, die im Wesentlichen halbkreisförmig ausgebildet und fest mit der ersten Branche 12 verbunden ist. Entlang des Außenrandes dieser Befestigungsplatte 56 erstreckt sich eine ebenfalls halbkreisförmige Schale 57. Die Schale 57 weist eine Öffnung 60 auf, durch die eine sichelförmige Stellscheibe 54 ins Innere der Halbblende 51 ragt. Mittels des Stellelements 52 lässt sich die Stellscheibe 54 derart betätigen, dass diese eine Kreisbewegung um das Zentrum der Irisblende 50 ausführt. Zwischen der Stellscheibe 54 und der Befestigungsplatte 56 sind mehrere Lamellen 53, 53', 53" angeordnet, die abschnittsweise mit der Befestigungsplatte 56 drehbeweglich verbunden sind. Krallen 59, 59' an den Lamellen 53, 53', 53" greifen in entsprechende Lamellenkulissen 58 in der Stellscheibe 54 ein. Die Lamellen 53, 53', 53" sind also derart ausgebildet und angeordnet, dass die Ausrichtung der Lamellen 53, 53', 53" durch die Bewegung der Stellscheibe 54 veränderbar ist. Wie in den Fig. 8 und 9 gezeigt, können die Lamellen mindestens zwei Positionen einnehmen. Die Lamellen 53, 53', 53" haben Innenwände 66, die als Elektroden wirken. In einer ersten Position (Fig. 9) sind die Lamellen 53, 53' derart positioniert, dass sie kaum über die Stellscheibe 54 herausragen. Es bildet sich eine kreisförmige Durchgangsöffnung, in die die Hülse 32 hineinragt. Zwischen der Hülse 32 und den Innenrändern 66 der Lamellen 53, 53' befindet sich ein Spalt, der zur Aufnahme von Abschnitten der Hohlorgane geeignet ist.

In einer geschlossenen Position ist die Durchgangsöffnung derart verkleinert, dass die Innenränder 66 der Lamellen 53, 53', 53" auf der Hülse 32 aufliegen (vgl. Fig. 8). Soweit sich Gewebeabschnitte der Hohlorgane zwischen den Innenrändern der Lamellen 53, 53', 53" und der Hülse 32 befinden, werden diese mit einer vordefinierten Kraft aneinander und gegen die Hülse 32 gepresst. Diese Kraft lässt sich mit der Schließkrafteinstellvorrichtung 45 einstellen und wird über die Stellelemente 52, 52' auf die Lamellen 53, 53', 53" übertragen.

Die Abdeckungen 55, 55a sind wechselseitig an den Halbblenden 51, 51' angeordnet und schützen deren Mechanik.

Fig. 7 zeigt eine weitere Ausgestaltung der Lamellen 53, 53'. Diese sind gestuft ausgebildet und schließen sich derart, dass eine erste Lamelle 53 bei dem Schließvorgang nicht nur auf die zweite Lamelle 53' hinauf gleitet, sondern auch in diese greift.

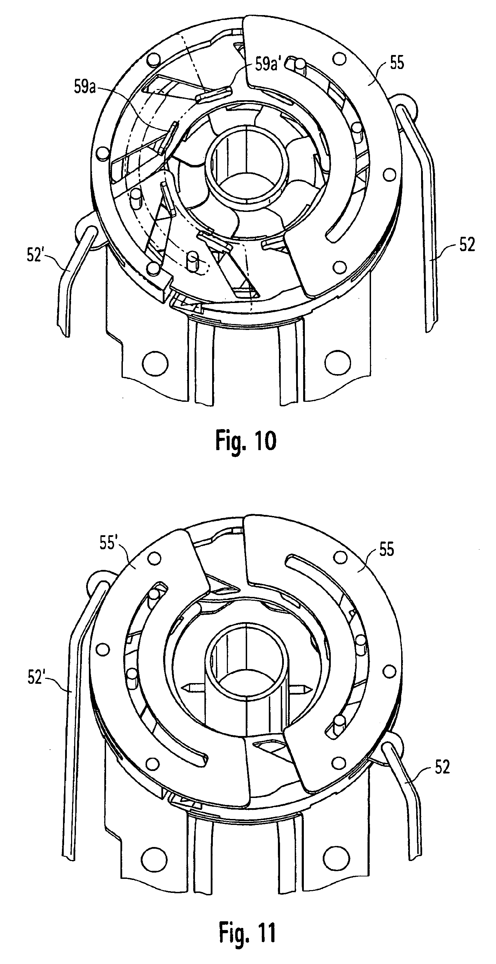

Eine weitere Ausführungsform der Lamellen 53, 53' lässt sich den Fig. 10 und 11 entnehmen. Die Fig. 10 und 11 zeigen die erfindungsgemäße Irisblende 50 in der Draufsicht, wobei die Irisblende 50 in Fig. 10 geschlossen und in Fig. 11 geöffnet ist. Um eine bessere Ansicht auf die Mechanik der Irisblende 50 zu haben, ist die Abdeckung 55' in Fig. 10 entfernt. Es zeigt sich, dass die Lamellen 53, 53' über T-Anker 59a, 59a' in die Lamellenkulisse 58 der Stellscheibe 54 eingreifen. Die T-Anker 59a, 59a' übernehmen die Funktion der Krallen 59, 59'. Stifte, die auf der Stellscheibe 54 der Halbblenden 51, 51' angeordnet sind und in die Abdeckungen 55, 55' hineinragen, bilden eine Kulissenführung für die Stellscheiben 54. Sie führen die Stellscheiben 54 bei ihrer Rotationsbewegung um das Zentrum der Irisblende 50 und erhöhen dabei den Schließbereich der Lamellen.

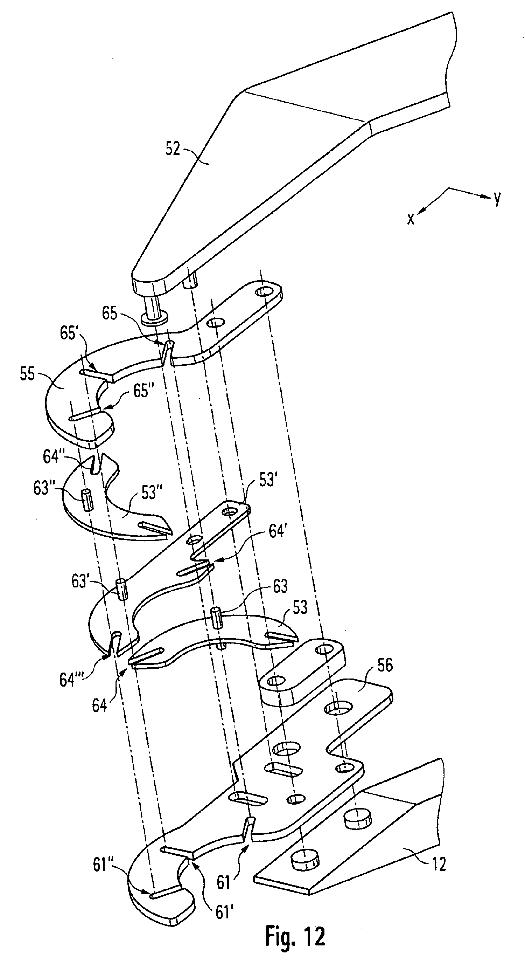

Die Fig. 12 bis 14 zeigen ein weiteres Ausführungsbeispiel der Mechanik der erfindungsgemäßen Irisblende 50 mit einfacherem Aufbau. Um die mechanischen Abläufe erläutern zu können, wird ein Koordinatensystem eingeführt. Die X-Achse dieses Koordinatensystems erstreckt sich im Wesentlichen entlang einer Branche 12. Die Y-Achse steht hierauf quer und zeigt auf das Zentrum der Durchgangsöffnung der Irisblende 50.

Die Fig. 12 zeigt beispielhaft die erste Halbblende 51 in einer Explosionszeichnung. Ein wesentlicher Unterschied des Ausführungsbeispieles gemäß den Fig. 12 bis 14 gegenüber dem der Fig. 6 besteht darin, dass die Funktion der Stellscheibe 54 in den Ausführungsbeispielen der Fig. 12 bis 14 durch eine Lamelle 53 bis 53", nämlich die Lamelle 53' wahrgenommen wird. Die Befestigungsplatte 56 ist gemäß der Fig. 12 ebenfalls mit der ersten Branche 12 fest verbunden. Sie umfasst drei Aussparungen 61, 61', 61", die radial in Richtung auf das Zentrum der Durchgangsöffnung der Irisblende 50 geöffnet sind. In diesen Aussparungen 61, 61', 61" lagern Lamellenstifte 63, 63', 63". Diese sind im Wesentlichen mittig auf den Lamellen 53, 53' 53" angeordnet. Die Aussparungen 61, 61', 61" bilden eine Kulissenführung entlang derer die einzelnen Lamellen 53, 53' 53" auf das Zentrum der Durchgangsöffnung zu bewegt werden können. Die Lamellenstifte 63, 63', 63" sind auf beiden Seiten der Lamellen 53, 53', 53" ausgebildet. Sie greifen auf der einen Seite in die Aussparungen 61, 61', 61" der Befestigungsplatte 56 ein und auf der anderen Seite der Lamellen 53, 53', 53" in Aussparungen 65, 65', 65" der Abdeckung 55. Die Aussparungen 65, 65', 65" der Abdeckungen 55 erstrecken sich ebenfalls im Wesentlichen auf das Zentrum der Durchgangsöffnung der Irisblende 50 zu. Die Lamelle 53' ist starr mit dem Stellelement 52 verbunden und weist hierfür einen entsprechenden Fortsatz auf. Das Stellelement 52 wird entlang der Y-Achse betätigt. Somit kann mittels des Stellelements 52 die zweite Lamelle 53' in Richtung auf das Zentrum der Durchgangsöffnung zu bewegt werden. Die halbkreisförmigen Lamellen 53 bis 53" weisen an ihren Enden jeweils eine Aussparung 64, 64', 64", 64"' auf, die in die Stifte 63, 63', 63" der anderen Lamellen 53 bis 53" eingreifen. Somit besteht eine mechanische Kupplung zwischen der ersten Lamelle 53 und der zweiten Lamelle 53' (Aussparung 64, 64'), die wiederum mit der dritten Lamelle 53" (Aussparung 64", 64"') verbunden ist. Durch die Bewegung der zweiten Lamelle 53' entlang der Y-Achse werden die erste und die dritte Lamelle 53, 53" betätigt und verschoben. Die Kulissenführungen sind ausgebildet, um jede der Lamellen 53 bis 53" auf das Zentrum der Durchgangsöffnung zu zu bewegen, wenn die zweite Lamelle 53' entlang der Y-Achse verschoben wird. Durch eine Gegenbewegung der zweiten Lamelle 53' lässt sich diese Bewegung umkehren. Die Irisblende 50 öffnet sich.

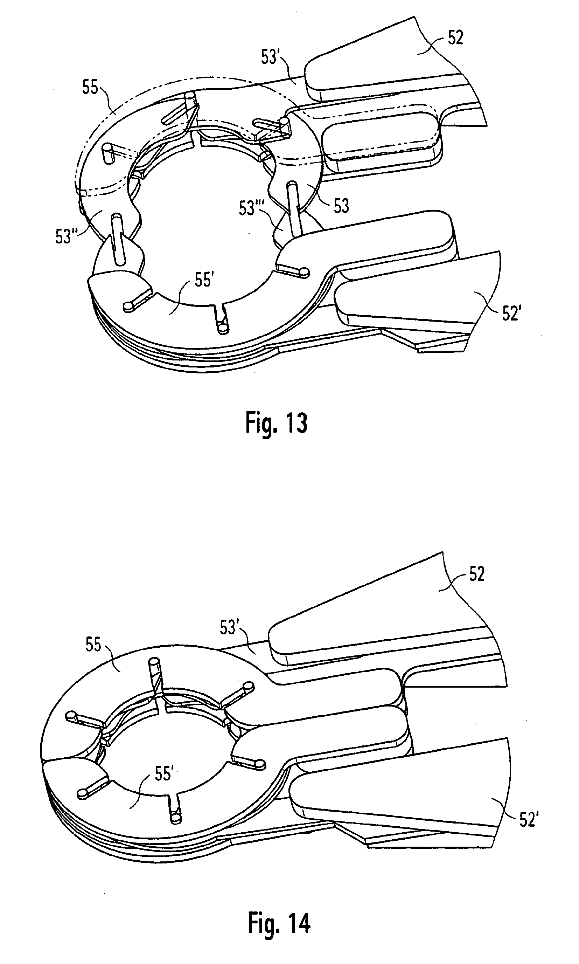

Die zweite Halbblende ist entsprechend symmetrisch aufgebaut. Die Fig. 13 und 14 zeigen die Halbblenden 51, 51'. Es zeigt sich, dass die erste Lamelle 53 der ersten Halbblende 51 in einem geschlossenen Zustand des elektrochirurgischen Instruments 10 eine mechanische Verbindung mit der ihr gegenüberliegenden Lamelle 53"" eingeht. Es besteht auch hier eine Verbindung zwischen den Lamellenstiften 63, 63', 63" und den Aussparungen 64, 64', 64". Das gleiche gilt für die dritte Lamelle 53".

Die Kupplung der Lamellen 53 bis 53" der ersten Halbblende 51 mit denen der zweiten Halbblende 51 ist lösbar. Im geöffneten Zustand des Instruments 10 hat die Irisblende 50 einen Spalt, durch den das verbundene Hohlorgan von dem Instrument 10 getrennt werden kann. Im geschlossenen Zustand des elektrochirurgischen Instruments 10 bildet sich also ein Ring aus Lamellen 53 bis 53"" aus, der gemäß dem Ausführungsbeispiel der Fig. 12 bis 14 sechs Lamellen umfasst. Die einzelnen Lamellen 53-53"' stehen jeweils mit den ihnen benachbarten Lamellen 53-53"' in Wirkverbindung. Es bildet sich insgesamt eine Kulissenführung aus, die eine Translationsbewegung der einzelnen Lamellen 53 bis 53"' in Richtung auf das Zentrum der Durchgangsöffnung zu ermöglicht. Die Betätigung der Stellelemente 52, 52' wird also mechanisch auf die einzelnen Lamellen 53 bis 53"' übertragen.

Erfindungsgemäß umfasst die Irisblende 50 eine erste Außenelektrodenanordnung oder Außenelektrode, die Hülse 32 eine Innenelektrodenanordnung, nämlich die Hülsenelektrode 36. Wie in der Fig. 5 gezeigt, sind diese Elektroden einander benachbart angeordnet. Die Abschnitte von Hohlorganen, die zwischen der Irisblende 50 und der Hülse 32 eingeklemmt sind, können mittels dieser Elektroden miteinander verbunden werden. Hierfür wird ein entsprechender HF-Strom an den Elektroden angelegt. Der HF-Strom führt zu einer Koagulation der Gewebeabschnitte, die sich zwischen den Elektroden befinden. Um den HF-Strom zu applizieren, weist das elektrochirurgische Instrument 10 entsprechende Leiterbahnen und Anschlüsse auf. Beispielsweise kann die Hülsenelektrode 36 über eine Leiterbahn innerhalb der Hülsenbetätigungsbranchen 55, 55' versorgt werden. Eine entsprechende Leiterbahn für die Irisblende kann sich innerhalb der Branchen 12, 12' befinden.

Andererseits können die Branchen 12, 12' und/oder die Hülsenbetätigungsbranchen 53, 53' ganz oder teilweise aus elektrisch leitendem Material ausgebildet sein, um eine entsprechende Leiterbahn bereitzustellen.

Die einzelnen Lamellen 53 bis 53"' können Elektroden zur Applikation des HF-Stroms umfassen und/oder aus elektrisch leitendem Material ausgebildet sein.

Vorzugsweise ist die Basisplatte 13 und der abnehmbare Abschnitt 13' der Basisplatte 13 ein elektrischer Isolator. Somit ist die Leiterbahn der Hülsenelektrode 36 zumindest von der Leiterbahn der Irisblende 50 elektrisch isoliert.

In einem weiteren Ausführungsbeispiel kann die Basisplatte 13 und/oder der abnehmbare Abschnitt 13' Anschlüsse und elektrische Leiterbahnen umfassen, um die Elektroden zu versorgen.

Es wurden Ausführungsbeispiele mit vier und acht Lamellen 53 bis 53"" beschrieben. Es sind weitere Ausführungsformen mit einer anderen Anzahl an Lamellen 53 bis 53"" denkbar (z. B. mit mindestens vier Lamellen 53 bis 53"").

### Bezugszeichenliste

- 10: Elektrochirurgisches Instrument
- 12, 12': Branchen
- 13: Basisplatte
- 13': Abnehmbarer Abschnitt der Basisplatte
- 20: Verschlussvorrichtung
- 24: Verschlussbetätigungselement
- 28: Dorn oder Fixierelement
- 30: Positionierungsvorrichtung
- 32: Hülse
- 33, 33': Hülsenabschnitt
- 35, 35": Hülsenbetätigungsbranche
- 36: Hülsenelektrode
- 40: Spannvorrichtung
- 41: Spannfeder
- 42: Spannvorrichtungsbetätigungselement
- 44: Kraftumlenker
- 45: Schließkrafteinstellvorrichtung
- 46: Stellschraube
- 47: Winkel
- 50: Irisblende
- 51, 51': Halbblende
- 52, 52': Stellelement
- 53 bis 53''': Lamelle
- 54: Stellscheibe
- 55, 55', 55a: Abdeckung
- 56: Befestigungsplatte
- 57: Schale
- 58: Lamellenkulisse in der Stellscheibe
- 59, 59': Kralle
- 59a, 59a': T-Anker
- 60: Öffnung
- 61, 61', 61": Aussparung in der Befestigungsplatte
- 63, 63', 63": Lamellenstift
- 64, 64', 64": Aussparung an der Lamelle
- 65, 65', 65": Aussparung in der Abdeckung
- 66: Innenrand

## Patentansprüche

1. Vorrichtung zur Herstellung von Anastomosen zwischen einem ersten und einem zweiten Hohlorgan, die jeweils eine Innen- und eine Außenfläche aufweisen, umfassend
- eine Hülse (32) mit einer Innenelektrodenanordnung (36), über welche das erste Hohlorgan so umgestülpt wird, dass seine Innenfläche nach außen zu liegen kommt, und
- eine Außenelektrodenanordnung (50), die der Innenelektrodenanordnung radial gegenüber liegt und mit dem zweiten Hohlorgan, welches über das erste Hohlorgan geschoben ist, so in elektrischen Kontakt bringbar ist, dass die Elektrodenanordnungen Außenflächen der Hohlorgane kontaktieren,
wobei
die Außenelektrodenanordnung (50) eine Vielzahl von durch mindestens ein Führungsorgan (52, 52') gegeneinander bewegbaren Lamellen (53-53"') umfasst, die derart ausgebildet und beweglich angeordnet sind, dass
- in einem Schließzustand Innenränder (66) der Lamellen (53 - 53"') eine Durchgangsöffnung bilden und am zweiten Hohlorgan anliegen und
- in einem Öffnungszustand ein von außen zur Durchgangsöffnung führender Spalt zwischen den Lamellen (53 - 53"') gebildet ist, durch den die miteinander verbundenen Hohlorgane aus der Durchgangsöffnung nach außen geführt werden können,
**dadurch gekennzeichnet daß** die Lamellen (53 - 53"') nach Art einer Irisblende (50) aufgebaut und angeordnet sind, um eine kreisförmige Durchgangsöffnung herzustellen.

2. Vorrichtung nach einem der vorhergehenden Ansprüche,
**dadurch gekennzeichnet, dass**
die Hülse (32) relativ zur Außenelektrodenanordnung (50) fixiert in einem Halter (34) angeordnet ist.

3. Vorrichtung nach Anspruch 2,
**dadurch gekennzeichnet, dass**
der Halter (34) zur individuellen Handhabung der Hülse (32) abnehmbar ist.

4. Vorrichtung nach einem der vorhergehenden Ansprüche,
**dadurch gekennzeichnet, dass**
alle Innenränder (66) der Lamellen (53 - 53"') im Schließzustand zur Hülse (32) einen gleichen Abstand aufweisen.

5. Vorrichtung nach einem der vorhergehenden Ansprüche,
**dadurch gekennzeichnet, dass**
die Durchgangsöffnung hinsichtlich ihres Durchmessers durch Bewegung der Lamellen (53) einstellbar ist.

6. Vorrichtung nach einem der vorhergehenden Ansprüche,
**dadurch gekennzeichnet, dass**
der Spalt im Öffnungszustand die Außenelektrodenanordnung (50) in zwei Abschnitte oder Hälften (51, 51') teilt, die voneinander weg bewegbar sind.

7. Vorrichtung nach einem der vorhergehenden Ansprüche,
**dadurch gekennzeichnet, dass**
die Hülse (32) an einer Betätigungseinrichtung (35, 35') gehalten und derart in zwei Teile zerlegbar ausgebildet ist, dass die Teile von einem Schließzustand zur Bildung eines geschlossenen Rohrabschnittes in einen Öffnungszustand zum Abnehmen vom Hohlorgan bringbar sind.

8. Vorrichtung nach Anspruch 7,
**dadurch gekennzeichnet, dass**
die Betätigungseinrichtung (35, 35') in den Schließzustand vorgespannt ist.

9. Vorrichtung nach einem der vorhergehenden Ansprüche,
**dadurch gekennzeichnet, dass**
die Lamellen (53 - 53"') durch eine Spanneinrichtung (40) mit einer definierten Kraft in den Schließzustand vorgespannt sind.

## Claims

1. Device for producing anastomoses between a first and a second hollow organ, each of the hollow organs having an inner surface and an outer surface, said device comprising
- a sleeve (32) with an inner electrode arrangement (36), over which the first hollow organ is turned such that the inner surface thereof comes to lie on the outside, and
- an outer electrode arrangement (50), which lies radially opposite the inner electrode arrangement and can be brought into electrical contact with the second hollow organ, which is pushed over the first hollow organ, such that the electrode arrangements contact outer surfaces of the hollow organs,
wherein the outer electrode arrangement (50) comprises a multiplicity of lamellae (53 - 53"'), which are movable relative to one another by at least one guide member (52, 52') and are designed and arranged movably in such a way that
- in a closed state, inner edges (66) of the lamellae (53 - 53"') form a through-opening and bear on the second hollow organ, and,
- in an open state, a gap leading from the outside to the through-opening is formed between the lamellae (53 - 53"'), through which gap the hollow organs connected to each other can be guided outwards from the through-opening,
**characterized in that** the lamellae (53 - 53"') are constructed in the manner of an iris diaphragm (50) and arranged to produce a circular through-opening.

2. Device according to the preceding claim, **characterized in that** the sleeve (32) is arranged fixed in a holder (34) relative to the outer electrode arrangement (50).

3. Device according to Claim 2, **characterized in that** the holder (34) is removable for individual manipulation of the sleeve (32).

4. Device according to one of the preceding claims, **characterized in that** all the inner edges (66) of the lamellae (53 - 53"') are at the same distance from the sleeve (32) in the closed state.

5. Device according to one of the preceding claims, **characterized in that** the through-opening is adjustable, in terms of its diameter, by movement of the lamellae (53).

6. Device according to one of the preceding claims, **characterized in that** the gap, in the open state, divides the outer electrode arrangement (50) into two sections or halves (51, 51'), which are movable away from each other.

7. Device according to one of the preceding claims, **characterized in that** the sleeve (32) is held on an actuating mechanism (35, 35') and is designed to be able to be dismantled in two parts, in such a way that the parts can be brought from a closed state, for formation of a closed tube section, to an open state, for removal from the hollow organ.

8. Device according to Claim 7, **characterized in that** the actuating mechanism (35, 35') is pre-tensioned in the closed state.

9. Device according to one of the preceding claims, **characterized in that** the lamellae (53 - 53"') are pre-tensioned in the closed state with a defined force by a tensioning device (40).

## Revendications

1. Dispositif pour produire des anastomoses entre un premier et un deuxième organe creux, lesquels présentent respectivement une surface extérieure et une surface intérieure, comprenant
- une douille (32), munie d'un arrangement d'électrode intérieur (36), sur laquelle le premier organe creux est retourné de telle sorte que sa surface intérieure se retrouve à l'extérieur, et
- un arrangement d'électrode extérieur (50), qui est opposé à l'arrangement d'électrode intérieur dans le sens radial et qui peut être amené en contact électrique avec le deuxième organe creux, lequel est emmanché sur le premier organe creux, de telle sorte que les arrangements d'électrode viennent en contact avec les surfaces extérieures des organes creux,
l'arrangement d'électrode extérieur (50) comprenant une pluralité de lamelles (53 - 53"') pouvant être déplacées les unes par rapport aux autres par le biais d'au moins un organe de guidage (52, 52'), lesquelles sont configurées et disposées avec mobilité de telle sorte que
- dans un état fermé, les bords intérieurs (66) des lamelles (53 - 53"') forment une ouverture de passage et reposent sur le deuxième organe creux et
- dans un état ouvert, il se forme un interstice entre les lamelles (53 - 53"') qui mène depuis l'extérieur vers l'ouverture de passage, à travers lequel les organes creux reliés ensemble peuvent être guidés vers l'extérieur hors de l'ouverture de passage,
**caractérisé en ce que** les lamelles (53 - 53"') sont constituées et disposées à la manière d'un diaphragme (50) afin de produire une ouverture de passage de forme circulaire.

2. Dispositif selon la revendication précédente, **caractérisé en ce que** la douille (32) est montée dans un support (34) en position fixe par rapport à l'arrangement d'électrode extérieur (50).

3. Dispositif selon la revendication 2, **caractérisé en ce que** le support (34) peut être retiré pour une manipulation individuelle de la douille (32).

4. Dispositif selon l'une des revendications précédentes, **caractérisé en ce que** tous les bords intérieurs (66) des lamelles (53 - 53"'), dans l'état fermé, présentent un écart identique par rapport à la douille (32).

5. Dispositif selon l'une des revendications précédentes, **caractérisé en ce que** le diamètre de l'ouverture de passage est réglable en déplaçant les lamelles (53).

6. Dispositif selon l'une des revendications précédentes, **caractérisé en ce que** dans l'état ouvert, l'interstice sépare l'arrangement d'électrode extérieur (50) en deux portions ou moitiés (51, 51') qui peuvent être déplacées pour s'éloigner l'une de l'autre.

7. Dispositif selon l'une des revendications précédentes, **caractérisé en ce que** la douille (32) est maintenue sur un dispositif d'actionnement (35, 35') et est configurée fractionnable en deux parties de telle sorte que les parties peuvent être amenées d'un état fermé pour former une portion tubulaire fermée en un état ouvert pour le retrait de l'organe creux.

8. Dispositif selon la revendication 7, **caractérisé en ce que** le dispositif d'actionnement (35, 35') est précontraint dans l'état fermé.

9. Dispositif selon l'une des revendications précédentes, **caractérisé en ce que** les lamelles (53 - 53"') sont précontraintes par un dispositif de serrage (40) avec une force définie dans l'état fermé.
